# EUROPÄISCHE PATENTSCHRIFT

(11) **EP 0 412 380 B1**
(45) Veröffentlichungstag und Bekanntmachung des Hinweises auf die Patenterteilung: **27.10.1993**
(21) Anmeldenummer: 90114531.8
(22) Anmeldetag: 28.07.1990
(51) Int. Cl.: C07C 265/14, C07C 263/16, C09D 175/04

(54) **Estergruppen aufweisende Polyisocyanate oder Polyisocyanatgemische, ein Verfahren zur Herstellung derartiger Polyisocyanate oder Polyisocyanatgemische und ihre Verwendung in Polyurethanlacken**
Polyisocyanates or mixtures of polyisocyanates with estergroups, a process for the preparation of such isocyanates or such mixtures of polyisocyanates and their utilization for polyurethane lacquers
Polyisocyanates ou mélanges de polyisocyanates contenant de groupes ester, un procédé pour la préparation de tels polyisocyanates ou tels mélanges de isocyanates et leur utilisation pour laques à base de polyuréthanes

(30) Priorität: 10.08.1989 DE 3926389
(43) Veröffentlichungstag der Anmeldung: 13.02.1991
(73) Patentinhaber: BAYER AG, 51368 Leverkusen (DE)
(72) Erfinder: Schmalstieg, Lutz, Dr., D-5000 Köln 1 (DE); Pedain, Josef, Dr., D-5000 Köln 80 (DE); Nachtkamp, Klaus, Dr., D-4000 Düsseldorf 13 (DE); Kahl, Lothar, Dr., D-5060 Bergisch Gladbach (DE); Schönfelder, Manfred, Dr., D-5090 Leverkusen 3 (DE)

(56) Entgegenhaltungen:
- DE-A- 1 913 273
- DE-A- 3 634 248

## Beschreibung

Die Erfindung betrifft Estergruppen aufweisende aliphatische Polyisocyanate mit besonders hoher Funktionalität bei gleichzeitig extrem niedriger Viskosität, ein Verfahren zur Herstellung von derartigen Polyisocyanaten bzw. Polyisocyanatgemischen und ihre Verwendung als Isocyanatkomponente in Zweikomponenten-Polyurethanlacken.

Höherfunktionelle Polyisocyanate mit aliphatisch gebundenen Isocyanatgruppen haben als Isocyanatkomponente für Zweikomponenten-Polyurethanlacke größte wirtschaftliche und technische Bedeutung erlangt. Diese "Lackpolyisocyanate" des Standes der Technik werden im allgemeinen durch Biuretisierung, Trimeriserung oder Urethanisierung von niedermolekularen Diisocyanaten, insbesondere Hexamethylendiisocyanat, hergestellt.

Verfahren zur Herstellung aliphatischer Polyisocyanate mit Biuretstruktur sind in großer Anzahl bekannt. Monomerenfreie, durch Biuretisierung hergestellte handelsübliche Lackpolyisocyanate besitzen im allgemeinen mittlere Funktionalitäten zwischen 3 und 4, wobei Produkte mit hoher, d.h. nahe an 4 liegender Funktionalität zwangsläufig eine höhere Viskosität besitzen als solche mit niedriger, d.h. nahe an 3 liegender Funktionalität.

Besonders niedrigviskose Biuretpolyisocyanate werden z.B. in US-PS 3 903 127 beschrieben. Die Abhängigkeit zwischen Funktionalität und Viskosität demonstriert eindrucksvoll Beispiel 3 dieser Veröffentlichung. Das Polyisocyanat mit der Funktionalität 3 besitzt eine Viskosität von 750 mPa.s/20°C (Bsp. 3e). Ist im Gemisch ein Anteil von 18 % an höherfunktionellen Produkten enthalten, so steigt die Viskosität auf 1350 mPa.s/20°C (Bsp. 3b). Steigt der Anteil höherfunktioneller Produkte auf 28 %, so besitzt das Gemisch eine Viskosität von 2560 mPa.s/20°C (Bsp. 3a).

Bei den Isocyanuratgruppen aufweisenden Polyisocyanaten, deren Herstellung ebenfalls in zahlreichen Veröffentlichungen beschrieben wird, liegen die Verhältnisse ähnlich. Die im allgemeinen eine mittlere Funktionalität von 3 bis 4 aufweisenden Polyisocyanate weisen bei vergleichbarer Funktionalität eine etwas niedrigere Viskosität als die Biuretgruppen aufweisenden Polyisocyanate auf. In der DE-OS 3 810 908 werden beispielsweise besonders niedrigviskose, Isocyanuratgruppen aufweisende Polyisocyanate beschrieben: Enthält die Oligomerenmischung einen Anteil von 30 % an Produkten mit einer Funktionalität größer als 3, so beträgt die Viskosität 1300 mPa.s/25°C (Bsp. 1). Enthält die Mischung einen Anteil an Produkten mit einer Funktionalität größer als 3 von 25 %, so beträgt die Viskosität nur 1000 mPa.s/25°C (Bsp. 2).

Die Herstellung von Lackpolyisocyanaten durch Urethanisierung niedermolekularer Diisocyanate mit mehrwertigen Alkoholen liefert hochviskose bis harzartige Produkte, die nur unter Zusatz von organischen Lösungsmitteln zu verarbeiten sind.

Wesentlich niedrigere Viskositäten besitzen nur Polyisocyanate, die einen großen Anteil an Uretdionstrukturen aufweisen, wie sie z.B. in US-PS 4 614 785 beschrieben werden. Diese Polyisocyanate weisen jedoch im allgemeinen eine unter 3 liegende mittlere Funktionalität auf, so daß die niedrige Viskosität mit dem Nachteil einer niedrigen Funktionalität erkauft werden muß.

Andererseits ist die mittlere NCO-Funktionalität bezüglich der Eignung der Polyisocyanate zur Herstellung von hochwertigen Lacküberzügen von großer Bedeutung: Je höher die Funktionalität, desto höher ist die Vernetzungsdichte im Lackfilm und damit dessen Widerstandsfähigkeit. Die mit den hohen Funktionalitäten verbundenen höheren Viskositäten sind jedoch andererseits nachteilig, da sie einen vermehrten Einsatz organischer Lösungsmittel zur Einstellung der erforderlichen Applikationsviskositäten notwendig machen.

Es war daher die der Erfindung zugrundeliegende Aufgabe, neue Lackpolyisocyanate bereitzustellen, die bei hoher Funktionalität gleichzeitig eine niedrige Viskosität aufweisen, so daß sie zur Herstellung von hochwertigen, lösungsmittelarmen oder -freien Zweikomponenten-Polyurethanlacken verwendet werden können.

Diese Aufgabe konnte mit der Bereitstellung der nachstehend näher beschriebenen Polyisocyanate bzw. Polyisocyanatgemische gelöst werden. Die neuen erfindungsgemäßen Lackpolyisocyanate weisen bei einer mittleren Funktionalität von mindestens 4,1, vorzugsweise mindestens 4,5 und besonders bevorzugt von mindestens 5 und eine Viskosität von maximal 2500 mPa.s/22°C, vorzugsweise von maximal 1000 mPa.s/22°C auf.

Von der chemischen Struktur her gesehen, handelt es sich bei den neuen Polyisocyanaten um Estergruppen aufweisende Polyisocyanate eines unter 2000 liegenden mittleren Molekulargewichts und einer mittleren Funktionalität von mindestens 4,1.

Estergruppenhaltige Polyisocyanate sind schon früher bekannt geworden. So sind beispielsweise in DE-OS 2 120 090 estergruppenhaltige Polyisocyanat-Prepolymere mit mittleren Molokulargewichten von ca. 900 bis 20 000 beschrieben, die sich zur Ausrüstung von Textilien eignen. Diese Produkte werden aus verdünnten Lösungen angewendet. Zur Formulierung lösungsmittelarmer Zweikomponentenlacke sind sie nicht geeignet. Weiterhin ist in DE-OS 3 634 248 ein Verfahren zur Herstellung estergruppenhaltiger Polyisocyanate beschrieben, das sich im Prinzip auch zur Herstellung der erfindungsgemäßen Poly isocyanate eignet. Allerdings werden in der DE-OS 3 634 248 hochfunktionelle Polyisocyanate der erfindungsgemäßen Art bzw. ihre Herstellung nicht beschrieben, da zur Herstellung der Estergruppen aufweisenden Polyisocyanate lediglich Alkohole mit einer maximalen Funktionalität von 4 zu Polyisocyanaten umgesetzt werden, die destillierte, analysenreine Verbindungen darstellen, deren Funktionalität der Funktionalität der ihnen zugrundeliegenden mehrwertigen Alkohole entspricht. Polyisocyanatgemische auf Basis von 4-wertigen Alkoholen, die neben den entsprechenden tetrafunktionellen Polyisocyanaten höherfunktionelle Homologe aufweisen und daher eine mittlere Funktionalität von mindestens 4,1 aufweisen, sind in der Vorveröffentlichung ebensowenig beschrieben wie ein Verfahren zu ihrer Herstellung, ganz zu schweigen von einem Verfahren zur Herstellung von hochfunktionellen, niedrigviskosen, Estergruppen aufweisenden Polyisocyanaten auf Basis von fünf- oder höherwertigen Polyolen.

Gegenstand der Erfindung sind durch Umsetzung von Isocyanatocarbonsäurechloriden mit O-silylierten, 4- bis 8-wertigen Alkoholen erhältliche, Estergruppen aufweisende, Polyisocyanate oder Polyisocyanatgemische, gekennzeichnet durch,
a) einen Gehalt an aliphatisch gebundenen Isocyanatgruppen von 18 bis 33 Gew.-%,
b) eine (mittlere) NCO-Funktionalität von 4,1 bis 10, vorzugsweise 4,5 bis 10 und
c) eine Viskosität von 200 bis 2500 mPa.s bei 22°C.

Gegenstand der Erfindung sind insbesondere auch die besonders bevorzugten derartigen Estergruppen aufweisenden Polyisocyanate oder Polyisocyanatgemische, die durch Umsetzung von Isocyanatocarbonsäurechloriden mit O-silylierten 5 und/oder 6 Hydroxylgruppen aufweisenden Zucker oder Zuckeralkoholen zugänglich sind, gekennzeichnet durch
a) einen Gehalt an aliphatisch gebundenen Isocyanatgruppen von 20 bis 33 Gew.-%,
b) eine (mittlere) NCO-Funktionalität von 5 bis 8 und
c) eine Viskosität von 500 bis 1000 mPa.s bei 22°C.

Gegenstand der Erfindung ist auch ein Verfahren zur Herstellung der erfindungsgemäßen, Estergruppen aufweisenden Polyisocyanate durch Umsetzung von Isocyanatocarbonsäurechloriden der Forrnel

OCN-R-COCl

in welcher
- R: für einen gesättigten aliphatischen Kohlenwasserstoffrest mit 2 bis 5 Kohlenstoffatomen steht,

mit O-silylierten, mehrwertigen Alkoholen bei 50 bis 150° C unter destillativer Entfernung des sich bildenden trisubstituierten Chlorsilans, dadurch gekennzeichnet, daß man (i) als O-silylierte mehrwertige Alkohole 4 bis 8 Hydroxylgruppen aufweisende Alkohole verwendet, in denen alle Hydroxylgruppen in silylierter Form vorliegen und (ii) bei der Verwendung von 4-wertigen Alkoholen einen Überschuß von Chlorcarbonylgruppen gegenüber silylierten Hydroxylgruppen vermeidet und eine Destillation des Verfahrensprodukts unterläßt.

Gegenstand der Erfindung ist schließlich auch die Verwendung der erfindungsgemäßen Polyisocyanate oder Polyisocyanatgemische als Isocyanatkomponente in Zweikomponenten-Polyurethanlacken.

Die bevorzugten erfindungsgemäßen Polyisocyanate oder Polyisocyanatgemische weisen eine mittlere NCO-Funktionalität von mindestens 4,5 auf. Dies gilt auch für erfindungsgemäße Polyisocyanatgemische auf Basis von 4-wertigen Alkoholen. Die erfindungsgemäß besonders bevorzugten Polyisocyanate oder Polyisocyanatgemische basieren auf 5- und/oder 6-wertigen Alkoholen und werden nach dem erfindungsgemäßen Verfahren hergestellt.

Grundsätzlich erfolgt die Herstellung der erfindungsgemäßen Polyisocyanate oder Polyisocyanatgemische durch Umsetzung von Isocyanatocarbonsäurechloriden mit O-silylierten mehrwertigen Alkoholen, wobei bei der Durchführung des erfindungsgemäßen Verfahrens zur Herstellung der besonders bevorzugten erfindungsgemäßen Polyisocyanate oder Polyisocyanatgemische Zucker und/oder Zuckeralkohole mit 5 und/oder 6 Hydroxylgruppen pro Molekül eingesetzt werden, deren Hydroxylgruppen ausschließlich in silylierter Form vorliegen.

Als Isocyanatocarbonsäurechloride kommen insbesondere Verbindungen der allgemeinen Formel

OCN - R - COCl

in Betracht, in welcher
- R: für einen zweiwertigen, gesättigten aliphatischen Kohlenwasserstoffrest mit 2 bis 5 Kohlenstoffatomen steht, wobei zwischen der Isocyanatgruppe und der Chlorcarbonylgruppe mindestens 2 Kohlenstoffatome angeordnet sind.

Gut geeignet sind beispielsweise 3-Isocyanatopropionsäurechlorid, 4-Isocyanatobuttersäurechlorid oder 6-Isocyanatocapronsäurechlorid.

Unter "O-silylierten Hydroxylgruppen" sind im Rahmen der Erfindung Struktureinheiten der Formel

R₃'Si-O-

zu verstehen, wobei
- R': für einen Alkyl- oder Arylrest, vorzugsweise eine C₁-C₄-Alkylgruppe und besonders bevorzugt für eine Methylgruppe steht.

Als Reaktionspartner für die Isocyanatocarbonsäurechloride kommen bei der Herstellung der erfindungsgemäßen Polyisocyanate oder Polyisocyanatgemische 4- bis 8-wertige, vorzugsweise 5- und/oder 6-wertige Alkohole in Betracht, deren Hydroxylgruppen in silylierter Form vorliegen. Geeignete mehrwertige Alkohole zur Herstellung dieser Vorprodukte sind beispielsweise Pentaerythrit, Mannit, Sorbit, Formit, Fruktose, Glukose, Saccharose, Lactose oder beliebige andere Zucker oder Zuckeralkohole mit 4 bis 8 Hydroxylgruppen pro Molekül. Auch beliebige Gemische derartiger mehrwertiger Alkohole können verwendet werden.

Die Herstellung der silylierten Ausgangsmaterialien aus diesen mehrwertigen Alkoholen erfolgt dabei nach beliebigen Methoden wie sie beispielsweise beschrieben sind in M. Lalonde und C.H. Chan, Synthesis 1985, S. 817 - 845. Geeignet zur Silylierung der Polyhydroxylverbindungen sind z.B. Chlorsilane und/oder Disilazane der allgemeinen Formel 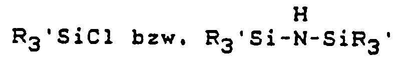
worin
- R': die obengenannte Bedeutung hat.

Die Natur des Restes R' ist jedoch für die Herstellung der erfindungsgemäßen Polyisocyanate von untergeordneter Bedeutung.

Die Silylierung von Zuckeralkoholen wurde beispielsweise von M. M. Sprung und L.S. Nelson in J. Org. Chem. 20, S. 1750 (1955) und die Silylierung von Zuckern von F. A. Henglein und K. Scheinost in Makromol. Chem. 21, S. 59 (1956) beschrieben.

Zur Herstellung der Polyisocyanate werden die Mengen an Isocyanatocarbonsäurechlorid und Silylether im allgemeinen so gewählt, daß auf jedes Mol Chlorcarbonylgruppen 1,0 bis 1,2 Mol silylierte Hydroxylgruppen entfallen. Vorzugsweise wird unter Einhaltung von äquimolaren Mengen der Einsatzstoffe gearbeitet. Die Umsetzung der silylierten Hydroxylverbindungen mit Isocyanatosäurechloriden erfolgt im allgemeinen im Temperaturbereich von 50 - 150°C, vorzugsweise 60 - 100°C.

Gegebenenfalls können die für diese Reaktion bekannten Katalysatoren wie z.B. Pyridin oder Chinolin zugesetzt werden.

Die Reaktion kann auch in Anwesenheit inerter Lösungsmittel durchgeführt werden, jedoch ist die Verfahrensweise ohne Mitverwendung von Lösungsmitteln besonders bevorzugt. Als Nebenprodukt entsteht das den silylierten Hydroxylgruppen entsprechende trisubstituierte Chlorsilan, vorzugsweise Trimethylchlorsilan, das sich destillativ aus dem Reaktionsgemisch entfernen läßt und vorzugsweise während der Umsetzung kontinuierlich abdestilliert wird.

Wegen der bevorzugten Arbeitsweise, derzufolge ein Überschuß von Chlorcarbonylgruppen gegenüber silylierten Hydroxylgruppen vermieden wird, entstehen bei der Umsetzung im wesentlichen chlorfreie, Estergruppen aufweisende Polyisocyanatgemische mit den bereits oben genannten Eigenschaften, die ohne weitere destillative Aufarbeitung der erfindungsgemäßen Verwendung zugeführt werden können. Es handelt sich im allgemeinen um Polyisocyanatgemische, deren gelchromatographische Analyse das gleichzeitige Vorliegen von Polyisocyanaten einer dem eingesetzten Alkohol entsprechenden Funktionalität als Hauptkomponente und von Polyisocyanaten des doppelten und dreifachen Molekulargewichts dieser Hauptkomponente aLs Nebenkomponenten aufzeigt. Die aus Isocyanatgehalt und dampfdruckosmometrisch bestimmtem Molekulargewicht errechenbare mittlere Funktionalität liegt daher auch bei Verwendung von vierwertigen Alkoholen stets bei mindestens 4,1, vorzugsweise bei mindestens 4,5.

Auch bei Einsatz von fünf- und höherwertigen Alkoholen, wird vorzugsweise wie oben angegeben verfahren. Dies bedeutet, es wird vorzugsweise ebenfalls darauf geachtet, daß bei der Umsetzung maximal äquivalente Mengen an Isocyanat und Carbonsäurechlorid, bezogen auf die silylierten Hydroxylgruppen, zum Einsatz gelangen, so daß als Umsetzungsprodukte praktisch chlorfreie Polyisocyanatgemische anfallen, die eine mittlere Funktionalität aufweisen, die über der Funktionalität des eingesetzten Alkohols liegt, und die undestilliert der erfindungsgemäßen Verwendung zugeführt werden können. Es ist jedoch auch möglich, nach der Durchführung des erfindungsgemäßen Verfahrens unter Verwendung von fünf- und/oder sechswertigen Alkoholen der beispielhaft genannten Art in O-silylierter Form die anfallenden Umsetzungsprodukte beispielsweise durch Hochvakuumdestillation aufzuarbeiten, so daß als Destillat die vorstehend bereits angesprochene Hauptkomponente anfällt, deren Funktionalität der Funktionalität des eingesetzten Alkohols entspricht. Bei einer derartigen destillativen Aufarbeitung der Reaktionsgemische können selbstverständlich auch höher als äquimolare Mengen an Isocyanatocarbonsäurechlorid, bezogen auf silylierte Alkohole, d.h., die bis zu 1,2-fach äquivalente Menge zur Einsatz gelangen. Höhere Überschüsse wären zwar prinzipiell nicht unmöglich, würden jedoch lediglich zu Ausbeuteverlusten führen.

Die besonders bevorzugten erfindungsgemäßen Polyisocyanate bzw. Polyisocyanatgemische, die nach dem erfindungsgemäßen Verfahren unter Verwendung von fünf- und/oder sechswertigen Alkoholen in O-silylierter Form anfallen, weisen diesen Ausführungen entsprechend, eine (mittlere) NCO-Funktionalität von fünf bis acht auf.

Die erfindungsgemäßen Polyisocyanate bzw. Polyisocyanatgemische zeichnen sich durch Monomerenfreiheit und außergewöhnlich niedrige Viskositäten aus. Durch Verwendung von Gemischen verschiedener silylierter Polyalkohole sind Polyisocyanate jeder gewünschten Funktionalität herstellbar. Mit diesen Eigenschaften sind die erfindungsgemäßen Polyisocyanate hervorragend geeignet für die Herstellung von lösungsmittelarmen bzw. -freien Zweikomponenten-Polyurethanlacken.

Bevorzugte Reaktionspartner für die erfindungsgemäßen Polyisocyanate bei der Herstellung von Polyurethanlacken sind die in der Polyurethanlacktechnik an sich bekannten Polyhydroxypolyester und -ether, Polyhydroxypolyacrylate und ggf. niedermolekularen, mehrwertigen Alkohole. Auch Polyamine, insbesondere in blockierter Form als Polyketimine oder Oxazolidine sind denkbare Reaktionspartner für die erfindungsgemäßen Produkte. Die Mengenverhältnisse, in welchen die erfindungsgemäßen Polyisocyanate und die genannten Reaktionspartner bei der Herstellung von Polyurethanlacken eingesetzt werden, werden im allgemeinen so gewählt, daß auf eine Isocyanatgruppe 0,8 - 3, vorzugsweise 0,9 - 1,1, die gegenüber Isocyanatgruppen reaktionsfähige Gruppen entfallen.

Zur Beschleunigung der Aushärtung können in bekannter Weise die in der Isocyanatchemie üblichen Katalysatoren verwendet werden, wie z.B. tert. Amine wie Triethylamin, Pyridin, Methylpyridin, Benzyldimethylamin, N,N-Dimethylaminocyclohexan, N-Methylpiperidin, Pentamethyldiethylentriamin, N,N'-Endoethylenpiperazin, N,N'-Dimethylpiperazin usw., Metallsalze wie Eisen(III)-chlorid, Zinkchlorid, Zink-2-ethylcaproat, Zinn(II)-2-ethylcaproat, Dibutylzinn(IV)-dilaurat, Molybdänglykolat usw.

Die die erfindungsgemäßen Polyisocyanate oder Polyisocyanatgemische enthaltenden Lacke ergeben Filme, die überraschend gut auf metallischem Untergrund haften, besonders lichtecht, wärmestabil und sehr abriebfest sind. Darüber hinaus zeichnen sie sich durch große Härte, Elastizität, sehr gute Chemikalienbeständigkeit, hohen Glanz, ausgezeichnete Wetterbeständigkeit und gute Pigmentierbarheit aus.

Solche Lacke können neben den wesentlichen Komponenten gegebenenfalls die in der Lacktechnologie üblichen Pigmente, Verlaufshilfsmittel, Füllstoffe usw. enthalten.

Die folgenden Beispiele erläutern die Erfindung. Ein Vergleich der Eigenschaften eines erfindungsgemäßen 2-Komponenten-Polyurethanlacks mit denen eines nicht erfindungsgemäßen Polyurethanlackes verdeutlicht insbesondere die erfindungsgemäß ermöglichte Erhöhung des Festkörpergehalts bei gleicher Viskosität. Alle Prozentangaben beziehen sich, soweit nichts anderes vermerkt auf das Gewicht.

### Beispiel 1

### Herstellung eines Polyisocyanatgemisches

424 g (1 Mol) 2,2-Bistrimethylsiloxymethyl-1,3-bis-trimethylsiloxypropan, hergestellt durch Silylierung von Pentaerythrit, gemäß M.M. Sprung, L.S. Nelson, J. Org. Chem. 20, S. 1750 (1955) und 702 g (4 Mol) 6-Isocyanatocapronsäurechlorid wurden unter Zusatz von 1 ml Pyridin bei 90 - 100°C gerührt, bis das IR-Spektrum keine Säurechloridbande mehr zeigt. Während der Reaktion wird entstehendes Trimethylchlorsilan laufend abdestilliert. Nach Entfernen letzter Reste von Trimethylchlorsilan durch Dünnschichtdestillation erhält man ein Polyisocyanat mit folgenden Kenndaten:
NCO-Gehalt: 22,6 %
Viskosität bei 22°C: 210 mPa.s

Das dampfdruckosmometrisch bestimmte Molekulargewichtsmittel M̅w beträgt 880. Daraus berechnet sich eine Funktionalität von 4,73.

Das Gelchromatogramm zeigt 77 Flächen-% Produkt des theoretischen Molekulargewichts von 692 g und 15,3 % eines Produktes mit doppeltem Molekulargewicht und 7,7 % eines Produktes mit dreifachem Molekulargewicht.

### Beispiel 2

### Herstellung eines Polyisocyanatgemisches

614 g (1 Mol) Hexa-trimethylsiloxy-Mannit, hergestellt durch Silylierung von Mannit, gemäß M.M. Sprung, L.S. Nelson, J. Org. Chem. 20, S. 1750 (1955) und 1053,6 g (6 Mol) 6-Isocyanatocapronsäurechlorid wurden wie in Beispiel 1 beschrieben zur Reaktion gebracht. Nach Entfernen letzter Reste von Trimethylchlorsilan durch Dünnschichtdestillation erhält man ein Produkt mit folgenden Kenndaten:
NCO-Gehalt: 23,1 %
Viskosität bei 22°C: 750 mPa.s

Das dampfdruckosmometrisch bestimmte Molekulargewichtsmittel M̅w beträgt 1110. Daraus berechnet sich eine Funktionalität von 6,56.

Die gelchromatographische Analyse des Produktgemisches zeigt 81 Flächen-% Produkt mit dem theoretischen Molekulargewicht von 1016, 16 Flächen-% Produkt mit dem doppeltem Molekulargewicht und 3 Flächen-% Produkt mit dem dreifachem Molekulargewicht.

### Beispiel 3

### Herstellung eines Polyisocyanatgemisches

540 g (1 Mol) Penta-trimethylsiloxy-glucose, hergestellt gemäß F.A. Henglein, K. Scheinost, Makromol. Chem. 21, S. 59 (1956) und 877,5 g (5 Mol) 6-Isocyanatocapronsäurechlorid wurden wie in Beispiel 1 beschrieben zur Reaktion gebracht. Nach Entfernen letzter Reste Trimethylchlorsilan durch Dünnschichtdestillation erhält man ein Produkt mit folgenden Kenndaten:
NCO-Gehalt: 22,4 %
Viskosität bei 22°C: 800 mPa.s

### Beispiel 4

### Herstellung eines Polyisocyanatgemisches

614 g (1 Mol) Hexa-trimethylsiloxy-sorbit, hergestellt durch Silylierung von Sorbit gemäß M.M. Sprung, L.S. Nelson, J. Org. Chem. 20, S. 1750 (1955) und 801 g (6 Mol) 3-Isocyanatopropionsäurechlorid wurden wie in Beispiel 1 beschrieben zur Reaktion gebracht. Nach Entfernen letzter Reste Trimethylchlorsilan durch Dünnschichtdestillation erhält man ein Produkt mit folgenden Kenndaten:
NCO-Gehalt: 30,1 %
Viskosität bei 22°C: 890 mPa.s

### Beispiel 5

### Herstellung eines 2-Komponenten-Polyurethanlackes

100 Gew.-Teile einer Polyollösung, bestehend aus 42 Gew.-Teilen Polyacrylatpolyol A, 28 Gew.-Teilen Polyesterpolyol B und 30 Gew.-Teilen Xylol werden mit 40 Gew.-Teilen Polyisocyanatgemisch aus Beispiel 1, gelöst in 24 Gew.-Teilen Methoxypropylacetat vermischt (Äquivalentverhältnis von Isocyanatgruppen zu gegenüber Isocyanatgruppen reaktionsfähigen Gruppen = 1 : 1).
Festkörpergehalt: 67 %
Auslaufzeit: 25 sec (DIN-Cup 4)
Prüfergebnisse der Lackfilme:
Einbrennbedingungen: 30 min bei 120°C
Pendelhärte nach König, DIN 53 157: 185 sec
Erichsen-Tiefung nach DIN/ISO 1520: 10,0 mm

### Polyacrylatpolyol A

Copolymerisat mit einem Gehalt an Hydroxylgruppen von 5,45 % und an Carboxylgruppen von 1,2 % aus
38,8 Gew.-Teile Hydroxypropylmethacrylat
21,6 Gew.-Teile Styrol
21,6 Gew.-Teile Methylmethacrylat
16 Gew.-Teile Butylacrylat
2 Gew.-Teile Acrylsäure

### Polyesterpolyol B

Polyesterpolyol mit einem Hydroxylgruppengehalt von 4,85 %, hergestellt durch Umsetzung von
19,5 Gew.-Teile 2-Ethylhexansäure
41,2 Gew.-Teile Trimethylolpropan
28,5 Gew.-Teile Hexahydrophthalsäureanhydrid
10,8 Gew.-Teile Adipinsäure

### Beispiel 6 (Vergleich)

### Herstellung eines 2-Komponenten-Polyurethanlackes auf Basis eines Isocyanuratgruppen-haltigen Polyisocyanatgemisches

100 Gew.-Teile der Polyollösung aus Beispiel 5 werden mit 42 Gew.-Teilen eines auf Hexamethylendiisocyanat basierenden Polyisocyanurat-Polyisocyanats mit einem NCO-Gehalt von 21,5 % und einer Viskosität von 3000 mPa.s/22°C, (dampfdruckosmometrisch bestimmtes mittleres Molekulargewicht M̅w = 760, Funktionalität = 3,89) gelöst in 40 Gew.-Teilen Methoxypropylacetat vermischt (Aquivalentverhältnis von Isocyanatgruppen zu gegenüber Isocyanatgruppen reaktionsfähigen Gruppen = 1 : 1).
Festkörpergehalt: 61,5 %
Auslaufzeit: 25 sec (DIN-Cup 4)
Prüfergebnisse der Lackfilme:
Einbrennbedingungen: 30 min bei 120°C
Pendelhärte nach König, DIN 53 157: 191 sec
Erichsen-Tiefung nach DIN/ISO 1520: 10,2 mm

## Patentansprüche

1. Durch Umsetzung von Isocyanatocarbonsäurechloriden mit O-silylierten, 4- bis 8-wertigen Alkoholen erhältliche, Estergruppen aufweisende Polyisocyanate oder Polyisocyanatgemische, gekennzeichnet durch
a) einen Gehalt an eliphatisch gebundenen Isocyanatgruppen von 18 bis 33 Gew.-%,
b) eine (mittlere) NCO-Funktionalität von 4,1 bis 10 und
c) eine Viskosität von 200 bis 2500 mPa.s bei 22° C.

2. Durch Umsetzung von Isocyanatocarbonsäurechloriden mit 5- und/oder 6-wertigen Zucker oder Zuckeralkoholen erhältliche, Estergruppen aufweisende Polyisocyanate oder Polyisocyanatgemische gemäß Anspruch 1, gekennzeichnet durch
a) einen Gehalt an aliphatisch gebundenen Isocyanatgruppen von 20 bis 33 Gew.-%,
b) eine (mittlere) NCO-Funktionalität von 5 bis 8 und
c) eine Viskosität von 500 bis 1000 mPa.s bei 22° C.

3. Verfahren zur Herstellung von Estergruppen aufweisenden Polyisocyanaten oder Polyisocyanatgemischen gemäß Anspruch 1 durch Umsetzung von Isocyanatocarbonsäurechloriden der Formel
OCN - R - COCl
in welcher
R für einen gesättigten aliphatischen Kohlenwasserstoffrest mit 2 bis 5 Kohlenstoffatomen steht,
mit O-silylierten, mehrwertigen Alkoholen bei 50 bis 150° C unter destillativer Entfernung des sich bildenden trisubstituierten Chlorsilans, dadurch gekennzeichnet, daß man (i) als O-silylierte mehrwertige Alkohole 4 bis 8 Hydroxylgruppen aufweisende Alkohole verwendet, in denen alle Hydroxylgruppen in silylierter Form vorliegen und (ii) bei der Verwendung von 4-wertigen Alkoholen einen Überschuß von Chlorcarbonylgruppen gegenüber silylierten Hydroxylgruppen vermeidet und eine Destillation des Verfahrensprodukts unterläßt.

4. Verfahren zur Herstellung von Estergruppen aufweisenden Polyisocyanaten oder Polyisocyanatgemischen gemäß Anspruch 2 durch Umsetzung von Isocyanatocarbonsäurechloriden der Formel
OCN - R - COCl
in welcher
R für einen gesättigten aliphatischen Kohlenwasserstoffrest mit 2 bis 5 Kohlenstoffatomen steht,
mit O-silylierten, mehrwertigen Alkoholen bei 50 bis 150° C unter destillativer Entfernung des sich bildenden trisubstituierten Chlorsilans, dadurch gekennzeichnet, daß man als O-silylierte mehrwertige Alkohole 5 und/oder 6 Hydroxylgruppen aufweisende Zucker oder Zuckeralkohole verwendet, in denen alle Hydroxylgruppen in silylierter Form vorliegen.

5. Verwendung der Estergruppen aufweisenden Polyisocyanate oder Polyisocyanatgemische gemäß Ansprüchen 1 und 2 als Isocyanatkomponente in Zweikomponenten-Polyurethanlacken.

## Claims

1. Ester-functional polyisocyanates or polyisocyanate mixtures obtainable by reaction of isocyanatocarboxylic acid chlorides with O-silylated 4- to 8-hydric alcohols, characterized by
a) a content of aliphatically bound isocyanate groups of 18 to 33% by weight,
b) an (average) NCO functionality of 4.1 to 10 and
c) a viscosity of 200 to 2,500 mPa.s at 22°C.

2. Ester-functional polyisocyanates or polyisocyanate mixtures obtainable by reaction of isocyanatocarboxylic acid chlorides with 5- and/or 6-hydric sugars or sugar alcohols alcohols, characterized by
a) a content of aliphatically bound isocyanate groups of 20 to 33% by weight,
b) an (average) NCO functionality of 5 to 8 and
c) a viscosity of 500 to 1,000 mPa.s at 22°C.

3. A process for the production of the ester-functional polyisocyanates or polyisocyanate mixtures claimed in claim 1 by reaction of isocyanatocarboxylic acid chlorides corresponding to the formula:
OCN - R - COCl
in which
R is a saturated, aliphatic hydrocarbon radical containing 2 to 5 carbon atoms,
with O-silylated, polyhydric alcohols at 50 to 150°C with removal of the trisubstituted chlorosilane formed by distillation, characterized in that (i) alcohols containing 4 to 8 hydroxyl groups, in which all the hydroxyl groups are present in silylated form, are used as the O-silylated polyhydric alcohols and (ii), where 4-hydric alcohols are used, an excess of chlorocarbonyl groups over silylated hydroxyl groups is avoided and the end product of the process is not distilled.

4. A process for the production of the ester-functional polyisocyanates or polyisocyanate mixtures claimed in claim 2 by reaction of isocyanatocarboxylic acid chlorides corresponding to the formula:
OCN - R - COCl
in which
R is a saturated, aliphatic hydrocarbon radical containing 2 to 5 carbon atoms,
characterized in that sugars or sugar alcohols containing 5 and/or 6 hydroxyl groups, in which all the hydroxyl groups are present in silylated form, are used as the O-silylated polyhydric alcohols.

5. The use of the ester-functional polyisocyanates or polyisocyanate mixtures claimed in claims 1 and 2 as isocyanate component in two-component polyurethane paints.

## Revendications

1. Polyisocyanates ou mélanges de polyisocyanates présentant des groupes ester, que l'on obtient par la mise en réaction de chlorures d'acides isocyanatocarboxyliques avec des alcools quadri- à octavalents O-silylés, qui se caractérisent par
a) une teneur en groupes isocyanate liés à des radicaux aliphatiques de 18 à 33% en poids,
b) une fonctionnalité NCO (moyenne) de 4,1 à 10, et
c) une viscosité de 200 à 2.500 mPa.s à 22°C.

2. Polyisocyanates ou mélanges de polyisocyanates présentant des groupes ester selon la revendication 1, que l'on obtient par la mise en réaction de chlorures d'acides isocyanatocarboxyliques avec des sucres ou des alcools de sucres penta- et/ou hexavalents, qui se caractérisent par
a) une teneur en groupes isocyanate liés à des radicaux aliphatiques de 20 à 33% en poids,
b) une fonctionnalité NCO (moyenne) de 5 à 8, et
c) une viscosité de 500 à 1.000 mPa.s à 22°C.

3. Procédé pour la préparation de polyisocyanates ou de mélanges de polyisocyanates présentant des groupes ester selon la revendication 1, par la mise en réaction de chlorures d'acides isocyanatocarboxyliques répondant à la formule
OCN-R-COCl
dans laquelle
R représente un radical d'hydrocarbure aliphatique saturé contenant de 2 à 5 atomes de carbone,
avec des alcools polyvalents O-silylés, à une température de 50 à 150°C avec élimination par distillation du chlorosilane trisubstitué qui se forme, caractérisé en ce qu'on utilise (i) comme alcools polyvalents O-silylés, des alcools présentant de 4 à 8 groupes hydroxyle, dans lesquels tous les groupes hydroxyle sont présents sous forme silylée et (ii) lors de l'utilisation d'alcools quadrivalents, on évite un excès de groupes chlorocarbonyle par rapport aux groupes hydroxyle silylés et on omet une distillation du produit issu du procédé.

4. Procédé pour la préparation de polyisocyanates ou de mélanges de polyisocyanates présentant des groupes ester selon la revendication 2, par mise en réaction de chlorures d'acides isocyanatocarboxyliques répondant à la formule
OCN-R-COCl
dans laquelle
R représente un radical d'hydrocarbure aliphatique saturé contenant de 2 à 5 atomes de carbone,
avec des alcools polyvalents O-silylés, à une température de 50 à 150°C avec élimination par distillation du chlorosilane trisubstitué qui se forme, caractérisé en ce qu'on utilise comme alcools polyvalents O-silylés, des sucres ou des alcools de sucres présentant 5 ou 6 groupes hydroxyle, dans lesquels tous les groupes hydroxyle sont présents sous forme silylée.

5. Utilisation des polyisocyanates ou des mélanges de polyisocyanates présentant des groupes ester selon les revendications 1 et 2, comme composant d'isocyanate dans des laques, des vernis ou des peintures de polyuréthanne à deux composants.
